(19) Europäisches Patentamt
European Patent Office
Office européen des brevets

(11) Numéro de publication: **0 409 712 A1**

(12) **DEMANDE DE BREVET EUROPEEN**

(21) Numéro de dépôt: **90402044.3**

(22) Date de dépôt: **16.07.90**

(51) Int. Cl.5: **C07D 303/48**, C07C 69/732, C07D 281/10

(30) Priorité: **18.07.89 FR 8909645**

(43) Date de publication de la demande: **23.01.91 Bulletin 91/04**

(84) Etats contractants désignés: **AT BE CH DE DK ES FR GB GR IT LI LU NL SE**

(71) Demandeur: **SANOFI**
**40, Avenue George V**
**F-75008 Paris(FR)**

(72) Inventeur: **Bousquet, André**
**Avenue du Lac**
**F-04200 Sisteron(FR)**
Inventeur: **Dormoy, Jean-Robert**
**Avenue du Thor 9**
**F-04200 Sisteron(FR)**
Inventeur: **Heymes, Alain**
**Rue F. Mistral 5**
**F-04200 Sisteron(FR)**

(74) Mandataire: **Le Guen, Gérard et al**
**CABINET LAVOIX 2, place d'Estienne d'Orves**
**F-75441 Paris Cédex 09(FR)**

(54) **Procédé de préparation d'un diastéréoisomère d'esters glycidiques, composés intermédiaires et procédé de préparation de dérivés de benzothiazépine-one.**

(57) L'invention a pour objet un procédé de préparation du diastéréoisomère trans(-)(2R,3S) des esters glycidiques de formule générale :

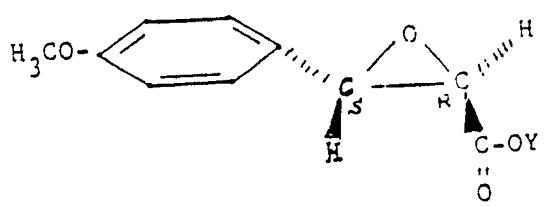

I

caractérisé en ce que l'on fait réagir une chlorhydrine de formule générale :

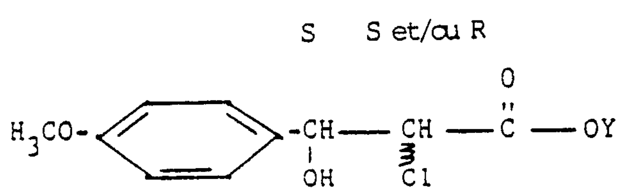

II

avec une base organique forte dans un solvant approprié et à une température comprise entre -10 °C et la température ambiante.

L'invention a également pour objet les composés intermédiaires de formule générale II ainsi qu'un procédé de préparation de cis(+)(2S,3S) benzothiazépine-1,5 one-4.

EP 0 409 712 A1

Le présente invention se rapporte, d'une manière générale, à un nouveau procédé de préparation d'esters glycidiques, à de nouveaux intermédiaires réactionnels ainsi qu'à un nouveau procédé de préparation de dérivés de benzothiazépine-one.

En particulier, l'invention concerne un nouveau procédé pour la préparation du diastéréoisomère trans(-)(2R,3S) des esters glycidiques de formule :

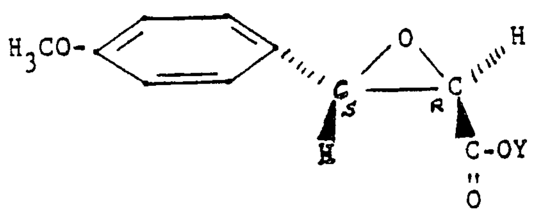

I

dans laquelle Y représente un radical alkyle en $C_1$-$C_8$.

Par radical "alkyle en $C_1$-$C_8$", on entend des restes d'hydrocarbures saturés linéaires ou ramifiés, tels que par exemple méthyle, éthyle, n-propyle, isopropyle, n-butyle, isobutyle, tertiobutyle, n-pentyle, n-hexyle, n-heptyle ou n-octyle.

De préférence, Y représente le radical méthyle.

Les diastéréoisomères trans(-)(2R,3S) de formule I constituent des composés intermédiaires particulièrement utiles pour la préparation notamment de l'acide cis(+)(2S,3S) hydroxy-2 (méthoxy-4 phényl)-3 (amino-2 phénylthio)-3 propionique.

Cet acide, décrit dans le brevet FR-A-2 530 243 est lui-même un intermédiaire de synthèse dont la mise en oeuvre, selon le procédé de brevet français en question, permet d'obtenir des dérivés diastéréo isomères cis(+)(2S,3S) de benzothiazépine-1,5 one-4 connus pour leurs propriétés antagonistes de la translocation calcique, hypotensives et vasodilatatrices coronariennes et cérébrales.

De tels dérivés peuvent être représentés par la formule générale :

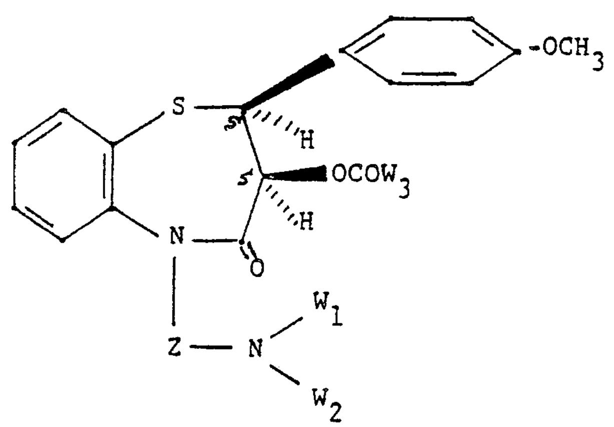

Ia

dans laquelle $W_1$, $W_2$ et $W_3$, qui sont identiques ou différents, représentent chacun un radical alkyle en $C_1$-$C_4$ de préférence méthyle et Z représente un radical alkylène en $C_1$-$C_4$, de préférence éthylène.

Le dérivé de benzothiazépine-1,5 one-4 le plus intéressant de cette série est incontestablement la cis-(+)(2S,3S)acétoxy-3 (N,N-diméthylamino-2 éthyl)-5(méthoxy-4 phényl)-2 dihydro-2,3 benzothiazépine (5H)-1,5 one-4 appelée communément diltiazem.

La plupart des composés connus actuellement pour la préparation de ce composé font appel à des synthèses non stéréospécifiques. Selon ces procédés, la configuration correcte des carbones 2 et 3 du diltiazem est recherchée par un dédoublement optique au niveau du composé final ou de l'un des intermédiaires au sein du procédé mis en oeuvre. Ainsi, on a envisagé le dédoublement de l'acide thréo-(2SR,3SR)hydroxy-2 (amino-2 phénylthio)-3 (méthoxy-4 phényl)-3 propionique au moyen par exemple de d-α-phényléthylamine (demande de brevet FR-A-2.530.243), de lysine (demande de brevet FR-A-2.534.579) ou encore de d-p-tolyléthylamine (demande de brevet JP-A-85/243.062).

De même, on a décrit le dédoublement de l'acide thréo(2SR,3SR) hydroxy-2 (nitro-2 phénylthio)-3-(méthoxy-4 phényl)-3 propionique et de l'acide thréo(2SR,3SR)hydroxy-2 (diméthylaminoéthylamino-2 phénylthio)-3 (méthoxy-4 phényl)-3 propionique au moyen de cinchonidine (demandes de brevet JP-A-78/018038 et JP-A-83/110685).

Enfin, on a effectué la séparation optique du diltiazem même au moyen d'acide mandélique optiquement actif (demande de brevet JP-A-83/032873) ou d'une colonne chirale (demande de brevet JP-A-85/046111).

Une telle étape de dédoublement optique influence défavorablement les rendements qui chutent, en conséquence, d'au moins 50%, le rendement final en diltiazem étant tout au plus de 25%.

En outre, ces séparations optiques augmentent sensiblement le prix de revient final du diltiazem car elles interviennent généralement au niveau de composés assez élaborés dans le cadre de la synthèse totale.

On a par ailleurs décrit dans les demandes de brevet JP-A-86/145159, JP-A-86/145160 et JP-A-86/145173, un procédé de préparation du diltiazem, à partir d'un ester glycidique(-) (2R,3S) obtenu par dédoublement optique de l'acide (+/-) (2RS,3SR) époxy-2,3 (méthoxy-4 phényl)-3 propionique avec une amine optiquement active de manière à récupérer un sel de métal alcalin de l'acide trans(-)(2R,3S) époxy-2,3 (méthoxy-4 phényl)-3 propionique que l'on estérifie en un ester(-) (2R,3S) époxy-2,3 (méthoxy-4 phényl)-3 propionique.

Ce procédé, comme les précédents, fait également appel à un dédoublement optique au niveau d'intermédiaires glycidiques.

En conséquence, il apparaît avantageux de pouvoir disposer d'un procédé industriel capable de fournir, selon une synthèse stéréospécifique, les esters trans(-) (2R,3S) époxy-2,3(méthoxy-4 phényl)-3 propionique de la demande de brevet JP-A-61/145160.

Une méthode largement connue et utilisée pour la préparation d'époxydes repose sur la réaction de Darzens qui comporte la cyclisation en milieu basique d'halohydrines obtenues à partir d'aldéhyde et d'un dérivé $\alpha$-halogéné.

Or, il est également bien établi que l'étape soit de rétroaldolisation soit de cyclisation des halohydrines produites au cours de cette réaction est très largement influencée par le milieu réactionnel et par les réactifs mis en présence de sorte qu'il s'avère impossible de prédire, avec certitude, le résultat final d'une telle réaction accomplie à partir d'une halohydrine quelconque et d'un milieu réactionnel basique imposé (J. Org. Chem., Vol. 34, No. 11, pp. 3600-3606).

Par exemple, on a décrit dans Tetrahedron Letters 27 pp. 5397-5400 (1986), une synthèse qui permet d'obtenir, en finalité un diastéréoisomère trans (2R,3S) d'ester glycidique. Selon ce procédé, on réalise l'époxydation selon la réaction de Darzens d'un mélange de chlorhydrines optiquement actives (2RS,3S), c'est-à-dire un mélange de (2RS,3S) chloro-2 hydroxy-3 butanoate d'éthyle pour obtenir un mélange 85/15 de trans/cis époxy-2,3 butanoate d'éthyle, la réaction étant effectuée en présence d'éthylate de sodium dans l'éthanol. Le rendement chimique de cette réaction s'est révélé être de 84%.

Après élimination de l'époxyde cis par passage transitoire au sel de brucine de l'acide butanoïque correspondant, l'acide trans (2R,3S) époxy-2 butanoïque optiquement pur (excès énantiomérique > 99%) a été recueilli avec un rendement de 37% seulement, ce qui laisserait supposer, compte tenu de la nature du sel, une résolution d'un mélange de diastéréoisomères trans.

Or, on a trouvé, selon l'invention, qu'il est possible, à partir de chlorhydrines sous forme de mélange de diastéréoisomères optiquement actifs (2RS,3S), d'obtenir des diastéréoisomères trans(-)(2R,3S) d'esters méthoxy-4 phénylglycidiques avec un rendement chimique très important, de manière très majoritaire puisque l'excès énantiomérique (ee) est supérieur à 85% et sans passage intermédiaire par la formation d'un sel.

Selon l'invention, on prépare les diastéréoisomères de formule I en faisant réagir une chlorhydrine de formule générale :

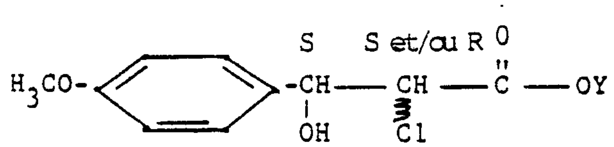

II

dans laquelle Y a la même signification que précédemment, avec un agent basique organique et dans un solvant approprié.

Comme base, on utilise généralement une base organique forte de préférence le 1,8-diazabicyclo [5,4,0] undec-7ène.

Le solvant utilisé est en général un solvant aprotique apolaire ou peu polaire tel que le dichloréthane, un solvant aprotique polaire tel que l'acétonitrile ou encore un solvant protique polaire tel qu'un alcool par

exemple le méthanol.

On préfère cependant utiliser un solvant polaire protique ou aprotique dans lequel la diastéréosélectivité de la transformation s'avère meilleure.

Quant à la réaction, celle-ci a lieu à une température allant de -10 °C à la température ambiante, de préférence à une température comprise entre -10 °C et +10 °C, qui permet une diastéréosélectivité maximale.

Les chlorhydrines de formule II peuvent être sous la forme soit d'un mélange de diastéréoisomères anti(+)(2S,3S) et syn(-)(2R,3S), soit du diastéréoisomère anti(+)(2S,3S), soit du diastéréoisomère syn(-)-(2R,3S).

Dans le cas particulier de mélanges d'anti(+)(2S,3S) et de syn(-) (2R,3S)chloro-2 hydroxy-3 (méthoxy-4 phényl)-3 propionates de méthyle, par exemple un mélange 61/39, on observe la formation, selon un rendement chimique pouvant atteindre 100% du seul ester glycidique trans qui se présente de manière très majoritaire sous la forme du diastéréoisomère trans(-)(2R,3S) puisque l'excès énantiomérique est de l'ordre de 86 à 88%.

Compte tenu de l'excès énantiomérique des chlorhydrines de départ, ce résultat témoigne d'une excellente conservation optique.

Ces résultats sont d'autant plus surprenants que des bromhydrines, analogues aux chlorhydrines de formule II, n'ont pu fournir qu'un mélange correspondant cis/trans d'époxydes de formule I, par conséquent, sans qu'aucune épimérisation ne soit intervenue.

On a observé, par ailleurs que la transformation par exemple en trans(-) (2R,3S) époxy-2,3 (méthoxy-4 phényl)-3 propionate de méthyle peut être réalisée en mettant en oeuvre l'anti(+)(2S,3S) chloro-2 hydroxy-3 (méthoxy-4 phényl)-3 propionate de méthyle, d'excès énantiomérique supérieur à 85% pour donner quantitativement et de manière univoque l'ester trans(-) (2R,3S) en question avec un excès énantiomérique de 80 à 85%.

De même, il est possible de préparer le même ester trans(-)(2R,3S) à partir du syn(-)(2R,3S) chloro-2 hydroxy-3 (méthoxy-4 phényl)-3 propionate de méthyle d'excès énatiomérique de 80 à 85%, pour donner avec un rendement de l'ordre de 90% l'ester trans(-)(2R,3S) en question avec un excès énantiomérique d'environ 85%.

Ces résultats mettent, par conséquent, en évidence :

- une rétention de la configuration absolue au niveau du carbone 3 des chlorhydrines de formule II, ce qui suppose une absence de rétrocondensation,
- la transformation, après épimérisation du carbone 2, de la chlorhydrine syn(-)(2R,3S) en l'ester glycidine trans(-)(2R,3S) au dépend de l'ester glycidique cis(2S,3S).

Ceci présuppose une cinétique suffisamment différenciée de cyclocondensation des deux chlorhydrines diastéréoisomères syn(-)(2R,3S) et anti(+)(2S,3S) et une cinétique d'épimérisation supérieure à celle de la cyclocondensation en époxyde cis.

Les chlorhydrines de formule II qu'elles soient sous forme de mélange(2R,3S) ou de diastéréoisomères séparés (2S,3S) et (2R,3S) constituent en conséquence des produits intermédiaires nouveaux particulièrement intéressants puisqu'ils pourront donner naissance avec facilité et grand rendement aux esters glycidiques de formule I eux-mêmes utiles pour la synthèse finale des composés de formule Ia et notamment du diltiazem.

Un second objet de l'invention consiste par conséquent en les chlorhydrines de formule II sous forme de diastéréoisomères anti(+)(2S,3S), de diastéréoisomères syn(-)(2R,3S) ou sous la forme de leurs mélanges, en tant que produits intermédiaires nouveaux utiles notamment pour la préparation finale des composés de formule Ia et notamment du diltiazem.

En particulier, l'invention concerne les chlorhydrines de formule II ci-dessous :

Anti(+)(2S,3S) chloro-2 hydroxy-3 (méthoxy-4 phényl)-3 propionate de méthyle

Syn(-)(2R,3S) chloro-2 hydroxy-3 (méthoxy-4 phényl)-3 propionate de méthyle,

ainsi que leurs mélanges.

Les chlorhydrines de formule II peuvent être obtenues de la manière suivante :

a) on fait réagir au reflux, dans un solvant approprié tel qu'un solvant aprotique par exemple le benzène, le toluène ou un xylène, et en présence d'un agent basique tel qu'un hydrure de métal alcalin, par exemple l'hydrure de sodium, la p-méthoxyacétophénone avec un composé de formule générale :

$$H_3CO-\overset{\overset{\displaystyle O}{\|}}{C}-OY \qquad III$$

dans laquelle Y a la même signification que précédemment, ce qui fournit un β-cétoester de formule générale :

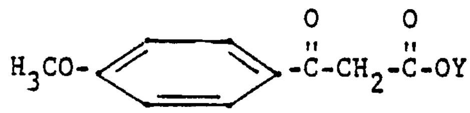

IV

dans laquelle Y a la même signification que précédemment,

b) on traite, alors, avec du chlorure de sulfuryle, le β-cétoester de formule IV à la température ambiante et dans un solvant approprié, par exemple un solvant aprotique tel que le benzène, le toluène ou un xylène, ce qui fournit un α-chloro β-cétoester de formule générale :

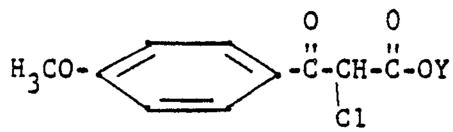

V

dans laquelle Y a la même signification que précédemment

c) on effectue ensuite une réduction stéréosélective de l'α-chloro β-cétoester de formule V selon la méthode décrite dans J. Chem. Soc. Chem. Commun. (1985), pp. 138-139 et J. Organometallic Chem. (1985) 290 -C23-C25, c'est-à-dire en traitant à une température comprise entre -70° C et -40° C, le composé de formule V en question, avec un mélange de (R,R) N,N′-dibenzoylcystine, de tertiobutanol et de borohydrure de lithium dans le tétrahydrofuranne, puis en décomposant le complexe formé par addition d'un acide fort tel que l'acide chorhydrique, ce qui fournit un mélange d'environ 50/50 de diastéréoisomères syn et anit de formule II très largement majoritaire en diastéréoisomère syn(-)(2R,3S) et anti(+)(2S,3S).

Si nécessaire, ce mélange de diastéréoisomères syn(-)(2R,3S) et anti(+)(2S,3S) peut être séparé en ses constituants selon des techniques connues par exemple pr chromatographie.

Quant à la (R,R) N,N′-dibenzoylcystine, celle-ci peut être préparée à partir de la (R)-cystine selon un protocole décrit dans J. Med. Chem. (1969) pp. 950-953.

Comme indiqué précédemment, les diastéréoisomères trans(-)(2R,3S) de formule I peuvent être utilisés pour la synthèse finale des dérivés cis(+)(2S,3S) de formule I et notamment du diltiazem.

Un troisième objet de l'invention est par conséquent un procédé de préparation des dérivés cis(+)-(2S,3S) de formule Ia au départ des diastéréoisomères trans(-)(2R,3S) de formule I eux-mêmes obtenus, selon l'invention, à partir des chlorhydrines de formule II.

A cet effet, le procédé consiste en les étapes suivantes :

a) on fait réagir le diastéréoisomère trans(-)(2R,3S) de formule I obtenu selon l'invention à partir des chlorhydrines de formule II, avec l'amino-2 thiophénol pour donner un ester cis(+)(2S,3S) hydroxy-2 (méthoxy-4 phényl)-3 (amino-2 phénylthio)-3 propionique de formule générale :

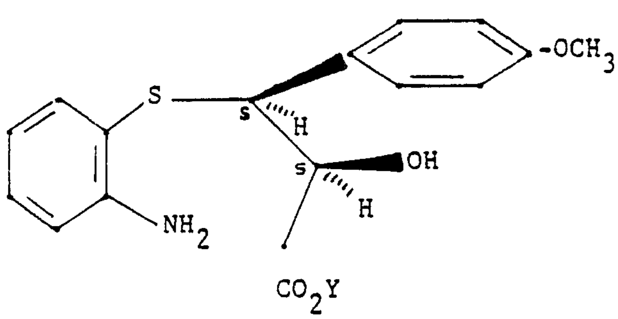

VI

dans laquelle Y a la même signification que précédemment,

b) on saponifie le composé de formule VI au moyen d'un agent basique tel qu'un hydroxyde de métal alcalin par exemple l'hydroxyde de sodium ou un carbonate de métal alcalin par exemple le carbonate de potassium, pour obtenir l'acide cis(+)(2S,3S) hydroxy-2 (méthoxy-4 phényl)-3 (amino-2 phénylthio)-3

propionique

c) on cyclise par chauffage à une température allant de 100 à 140 °C dans un hydrocarbure aromatique par exemple le toluène, un xylène ou l'éthylbenzène, l'acide cis(+)(2S,3S) hydroxy-2 (méthoxy-4 phényl)-3 (amino-2 phénylthio)-3 propionique ainsi obtenu, ce qui fournit la cis(+)(2S,3S) hydroxy-3 (méthoxy-4 phényl)-2 dihydro-2,3 benzothiazépine (5H)-1,5 one-4 de formule :

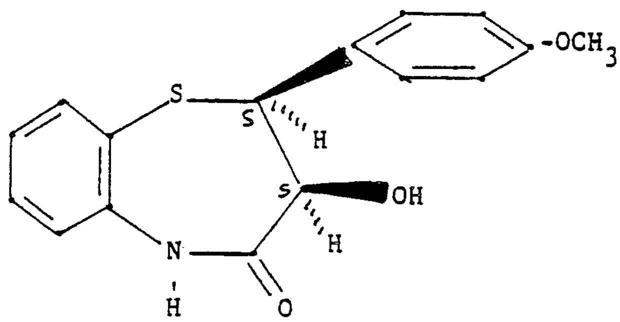

VII

d) on fait réagir le dérivé cis(+)(2S,3S) de formule VII avec un halogénure, de formule générale :

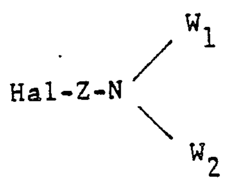

VIII

dans laquelle Hal représente un atome d'halogène par exemple le chlore et $W_1$, $W_2$ et Z ont la même signification que précédemment, ou un sel de cet halogénure, l'halogénure étant de préférence le chlore et le sel, le chlorhydrate, en présence d'un agent basique par exemple l'hydroxyde, le carbonate ou le bicarbonate de potassium et dans un solvant polaire par exemple le formamide, l'acétamide, le N,N-diméthylformamide ou le N,N-diméthylacétamide, pour obtenir les dérivés cis-(+)(2S,3S) de benzothiazépine-1,5 one 4 de formule générale :

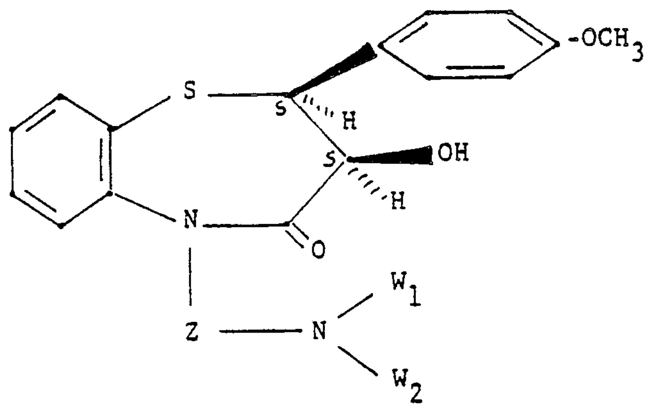

IX

dans laquelle $W_1$, $W_2$ et Z ont la même signification que précédemment,

e) on fait réagir, dans un solvant aprotique tel qu'un hydrocarbure aromatique au reflux, le dérivé cis(+)-(2S,3S) de benzothiazépine-1,5 one-4 de formule IX, avec un agent d'acylation approprié par exemple un anhydride de formule générale :

$$W_3-\overset{O}{\overset{\|}{C}}-O-\overset{O}{\overset{\|}{C}}-W_3 \qquad X$$

ou un acide de formule

$$W_3-\overset{O}{\overset{\|}{C}}-OH$$

dans lesquelles $W_3$ a la même signification que précédemment, pour obtenir les dérivés diastéréoisomères cis (+)(2S,3S) de benzothiazépine-1,5 one-4 de formule Ia sous forme de base libre, celle-ci étant mise en réaction, si nécessaire, avec un acide organique ou inorganique approprié pour former un sel pharmaceutiquement acceptable.

Les Exemples, non limitatifs suivants illustrent l'invention :

## PREPARATION

### Anti(+)(2S,3S) et syn(-)(2R,3S) chloro-2 hydroxy-3 (méthoxy-4 phényl)-3 propionates de méthyle.

#### a) (Méthoxy-4 phényl)-3 oxo-3 propionate de méthyle

Dans un réacteur de 2l muni d'un agitateur mécanique, d'un thermomètre, d'un réfrigérant et d'une ampoule d'introduction, on charge, sous atmosphère d'argon, 51g (1,064 mole) d'hydrure de sodium à 50% (préalablement lavé deux fois avec 100ml d'hexane), 340ml de toluène et 90ml (1,064 mole) de diméthylcarbonate.

On porte le milieu réactionnel au reflux puis on ajoute progressivement en 2 heures 80g (0,532 mole) de p-méthoxy-acétophénone en solution dans 180ml de toluène.

L'addition terminée, on maintient le milieu réactionnel durant 15 minutes au reflux, on refroidit puis on additionne, successivement 35ml d'acide acétique et 150ml d'eau (pH = 5). Après décantation et séparation, on extrait une nouvelle fois la phase aqueuse avec 80ml de toluène. On réunit les phases organiques, on les lave avec 50ml d'une solution saturée de bicarbonate de sodium, on sèche sur sulfate de sodium puis on évapore à siccité. On obtient ainsi une huile épaisse (54,3g) qui cristallise par addition d'un germe de (méthoxy-4 phényl)-3 oxo-3 propionate de méthyle.

De cette manière, on obtient le (méthoxy-4 phényl)-3 propionate de méthyle, avec un rendement de 98%, que l'on peut purifier après passage sur une colonne de silice.

P.F. : 40,3° C

Spectre de résonance magnétique nucléaire (R.M.N.) ($CDCl_3$) : 7,9 ppm (d,2H); 6,9 ppm (d,2H); 3,95 ppm (s,2H); 3,85 ppm (s,3H); 3,75 ppm (s,3H).

#### b) Chloro-2 (méthoxy-4 phényl)-3 oxo-3 propionate de méthyle

Dans un ballon de 250ml muni d'un agitateur, d'un thermomètre et d'un réfrigérant, on introduit, sous atmosphère d'argon, 24,96g (0,12 mole) de (méthoxy-4 phényl)-3 oxo-3 propionate de méthyle et 70ml de toluène anhydre. Sous agitation, on ajoute en 10 minutes, 10,19ml (0, 126 mole) de chlorure de sulfuryle, tout en maintenant la température à 20° C. Après 30 minutes d'agitation supplémentaires à 20° C, on additionne, au milieu réactionnel, 220ml d'une solution aqueuse saturée de bicarbonate de sodium (pH = 7) puis 50ml de toluène.

Après décantation et séparation, on sèche la phase toluénique sur sulfate de sodium puis on concentre sous vide.

De cette manière, on obtient 28,24g de chloro-2 (méthoxy-4 phényl)-3 oxo-3 propionate de méthyle, avec un rendement de 97%, que l'on peut purifier par passage sur une colonne de silice.

$n_D^{20}$ = 1,5630

Spectre R.M.N. ($CDCl_3$) : 8-7,95 ppm (d,2H); 6,95-6,90 ppm (d,2H); 5,60 ppm (s,1H); 3,9 ppm (s,3H); 3,8

ppm (s,3H).

c) Anti(+)(2S,3S) et syn(-)(2R,3S) chloro-2 hydroxy-3 (méthoxy-4 phényl)-3 propionate de méthyle.

Dans un ballon de 250ml muni d'un agitateur mécanique, d'un thermomètre, d'une ampoule d'introduction et d'un réfrigérant, on place, sous atmosphère d'argon, 2,87g (6,43 mmoles) de (R,R) N,N'-dibenzoylcystine, 32ml de tétrahydrofuranne anhydre, 0,634g de tertio-butanol en solution dans 32ml de tétrahydrofuranne anhydre et 19,3ml d'une solution 1M de borohydrure de lithium dans le tétrahydrofuranne.
Le milieu réactionnel hétérogène blanc s'homogénéise après chauffage au reflux pendant 3 heures. On refroidit ce dernier à -70 °C et on ajoute une solution de 1,3g(5,36 mmoles) de chloro-2 (méthoxy-4 phényl)-3 oxo-3 propionate de méthyle dans 11ml de tétrahydrofuranne en maintenant 1a température du milieu réactionnel à -70 °C. On laisse ensuite la température du milieu réactionnel remonter à -40 °C et on la maintient durant 2 heures. On ajoute alors 16,1ml d'acide chlorhydrique 1N, ce qui fait passer la température de -40 °C à 0 °C, puis 60ml d'eau et 80ml de dichlorométhane.
Après agitation vigoureuse du milieu, on décante et on extrait la phase aqueuse isolée avec 50ml de chlorure de méthylène.
On réunit les phases organiques puis on évapore à sec après sèchage sur sulfate de sodium pour obtenir 3,60g d'un produit pâteux que l'on purifie par filtration rapide sur silice.

De cette minière, on isole 1,02g de chloro-2 hydroxy-3 (méthoxy-4 phényl)-3 propionate de méthyle avec un rendement de 77,8%.
Diastéréoisomère syn/diastéréoisomère anti : 50/50

On a déterminé l'excès diastéréoisomérique du produit obtenu :

a) par chromatographie sur couche mince (dichlorométhane/acétate d'éthyle : 95/5) ou par chromatographie liquide à haute pression (C.L.H.P.)(détection 230 mn; débit 1,8 ml/min. éluant : eau/méthanol/acétonitrile/tétrahydrofuranne :770/50/180/10):
diastéréoisomère syn : 16,24 mn
diastéréoisomère anti : 25,56 mn

b) par R.M.N. du proton :

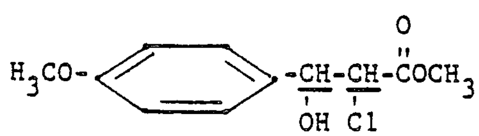

diastéréoisomère syn : 5,1 ppm (d,1H); 4,42 ppm (d,1H)
diastéréoisomère anti : 5 ppm (d,1H); 4,35 ppm (d,1H)

Les deux diastéréoisomères syn et anti ont été séparés par chromatographie liquide préparative à haute pression. L'excès énantiomérique des diastéréoisomères isolés a été déterminé sur le proton -C H -OH par R.M.N. du proton dans $C_6D_6$ après addition de Eu (tfc)$_3$. Leur pouvoir rotatoire a été déterminé par polarimétrie.

1) diastéréoisomère syn (-)(2R,3S)

R.M.N. : deux doublets se situant entre 5,1 et 4,85 ± 0,5 ppm
ee : 84%
$\alpha_D^{20}$ : -2° (c = 0,5, chloroforme)

2) diastéréosiomère anti(+)(2S,3S)

R.M.N. : deux doublets se situant entre 6,15 et 5,83 ± 0,5 ppm
ee : 88%
$\alpha_D^{20}$ : + 36° (c = 0,5, chloroforme)

## EXEMPLE 1

Préparation du trans(-)(2R,3S) époxy-2,3 (méthoxy-4 phényl)-3 propionate de méthyle.

Dans un ballon de 20ml muni d'un thermomètre, d'un réfrigérant et d'un agitateur mécanique, on introduit, sous atmosphère d'argon, 0,305g (1,25 mmole) d'anti(+)(2S,3S) chloro-2 hydroxy-3 (méthoxy-4 phényl)-3 propionate de méthyl obtenu précédemment ("Préparation") (ee = 88%), 2,5ml de dichloroéthane et 0,2ml (1,31 mmole) de 1,8-diazabicyclo[5,4,0] undec-7-ène. On obtient un milieu homogène.

Après 2 heures d'agitation à 20 °C, la réaction est terminée. On verse alors le milieu dans 10ml d'une solution tampon (pH = 7). Après addition de 10ml de dichloroéthane, extraction et décantation, on isole la phase organique et on évapore à siccité.

De cette manière, on obtient 0,250g de trans(-)(2R,3S) époxy-2,3 (méthoxy-4 phényl)-3 propionate de méthyle sous forme cristalline avec un rendement de 94,7%.

La présence d'époxyde cis n'a été décelée ni par R.M.N. ni par chromatographie liquide.

R.M.N. (dans diméthylsulfoxyde)

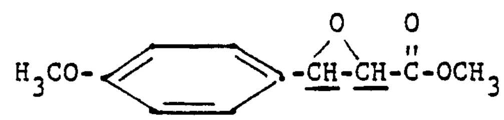

Diastéréoisomère trans : 4,1 ppm (d,1H); 3,84 ppm (d,1H)
Diastéréoisomère cis : 4,35 ppm (d,1H); 4 ppm (d,1H).

C. L. H. P.

a) Epoxyde cis : 22,28 mn
Epoxyde trans : 32,75 mn
(détection : 230 nm; éluant : eau 770, méthanol : 50, acétonitrile : 180, tétrahydrofuranne :10; débit : 1,8 ml/mn)
b) Par chromatographie en phase liquide sur une colonne chirale :
Eluant : isopropanol/hexane 50/50
Détection : 230 nm
Débit : 0,9 ml/mn
Epoxyde trans(-) : 24,1 mn
Epoxyde trans(+) : 20,6 mn on a déterminé l'excès énantiomérique du trans(-)(2R,3S) époxy-2,3 (méthoxy-4 phényl)-3 propionate de méthyle formé : 88%.

Pouvoir rotatoire

$\alpha_D^{20} = -133°$ (c = 0,892, chloroforme)

## EXEMPLE 2

Préparation du trans(-)(2R,3S) époxy-2,3 (méthoxy-4 phényl)-3 propionate de méthyle.

Dans un ballon de 20ml muni d'un thermomètre, d'un réfrigérant et d'un agitateur mécanique, on introduit, sous atmosphère d'argon, 0,305g (1,25 mmole) de syn(-)(2R,3S) chloro-2 hydroxy-3 (méthoxy-4 phényl)-3 propionate de méthyle obtenu précédemment ("Préparation") (ee = 84%), 2,5 ml de dichloroéthane et 0,2 ml (1,31 mmole) de 1,8-diazabicyclo[5,4,0] undec-7-ène.

On obtient un milieu homogène. Après 2 heures d'agitation à 20 °C, la réaction est terminée. On verse

alors le milieu dans 10ml d'une solution tampon (pH = 7). Après addition de 10ml de dichloroéthane, extraction et décantation, on isole la phase organique et on évapore à siccité. On obtient ainsi 0,260g (rendement : 96%) d'un produit cristallisé constitué, d'après la R.M.N. et la C.L.H.P. de 13% d'époxyde cis et de 87% d'époxyde trans.

Une séparation par C.L.H.P. permet d'isoler le trans(-)(2R,3S) époxy-2,3 (méthoxy-4 phényl)-3 propionate de méthyle pur.
ee : 84% (C.L.H.P.).

## EXEMPLE 3

Préparation du trans(-)(2R,3S) époxy-2,3 (méthoxy-4 phényl)-3 propionate de méthyle.

Dans un ballon de 50ml muni d'un thermomètre, d'un réfrigérant et d;un agitateur mécanique, on introduit, sous atmosphère d'argon, 0,78g de syn(-)(2R,3S) chloro-2 hydroxy-3 (méthoxy-4 phényl)-3 propionate de méthyle (ee = 70%), 1,22g d'anti(+)(2S,3S) chloro-2 hydroxy-3 (méthyoxy-4 phényl)-3 propionate de méthyle (ee = 100%), 4ml de dichloréthane et 1,32ml de 1,8-diazabicyclo[5,4,0]undec-7-ène. On obtient un milieu homogène. Après 2 heures d'agitation à 20 °C, la réaction est terminée. On verse le mélange réactionnel dans 50ml d'une solution tamponnée (pH = 7). Après addition de 40ml de dichloréthane, extraction de décantation, on isole la phase organique, sèche sur sulfate de sodium puis évapore à siccité pour obtenir une huile (1,72g) qui cristallise (rendement : 100%).

Ce produit est un mélange de trans(-)(2R,3S) époxy-2,3 (méthoxy-4 phényl)-3 propionate de méthyle et de cis (2S,3S) époxy-2,3 (méthoxy-4 phényl)-3 propionate de méthyle.

Sa composition diastéréoisomérique a été déterminée par R.M.N. du proton et par C.L.H.P.
Diastéréoisomère trans / diastéréoisomère cis : environ 95/5

Une séparation par C.L.H.P. permet d'isoler le trans(-)(2R,3S) époxy-2,3 (méthoxy-4 phényl)-3 propionate de méthyle pur. Diastéréoisomère trans(-) / diastéréoisomère trans(+) : 93,3 / 6,7 (ee = 86,6%) (C.L.H.P.)
$\alpha_D^{23}$ : -130 ° (c = 0,9, chloroforme)

## EXEMPLE 4

Préparation du trans(-)(2R,3S) époxy-2,3 (méthoxy-4 phényl)-3 propionate de méthyle.

Dans un ballon de 50ml muni d'un thermomètre et d'un agitateur méca nique, on introduit, sous atmosphère d'argon, 0,39g de syn(-)(2R,3S) chloro-2 hydroxy-3 (méthoxy-4 phényl)-3 propionate de méthyle (ee = 70%), 0,610 g d'anti (+)(2S,3S) chloro-2 hydroxy-3 (méthoxy-4 phényl)-3 propionate de méthyle (ee = 100%) et 2ml de méthanol. On refroidit le milieu à -10 °C et on y ajoute 0,66ml de 1,8-diazabicyclo[5,4,0]undec-7-ène. Après 4 heures d'agitation à -5 °C, on évapore le mélange réactionnel à siccité. On reprend le résidu huileux dans 25ml de dichloréthane et on lave avec 25ml d'une solution tamponnée (pH = 7). Après extraction et décantation, on isole la phase organique et on évapore à siccité pour obtenir 0,72g d'une huile qui cristallise (rendement : 85%).

Ce produit est un mélange de trans(-)(2R,3S) époxy-2,3 (méthoxy-4 phényl)-3 propionate de méthyle et de cis (2S,3S) époxy-2,3 (méthoxy-4 phényl)-3 propionate de méthyle. Sa composition diastéréoisomérique a été déterminée par R.M.N. du proton et par C.L.H.P.
Diastéréoisomère trans / diastéréoisomère cis : environ 99/1.

Une séparation par C.L.H.P. permet d'isoler le trans(-)(2R,3S) époxy-2,3 (méthoxy-4 phényl)-3 propionate de méthyle pur. Diastéréoisomère trans(-) / diastéréoisomère trans(+) : 94/6 (ee = 88%) $\alpha_D^{23}$ : -133 ° (c = 0,9, chloroforme)

## EXEMPLE 5

Préparation du chlorhydrate de cis(+)(2S,3S) acétoxy-3 (N,N-diméthylamino-2 éthyl)-5 (méthoxy-4 phényl)-2 dihydro-2,3 benzothiazépine (5H)-1,5 one-4 ou chlorhydrate de diltiazem.

a) Cis(+)(2S,3S) hydroxy-2 (amino-2 phénylthio)-3 (méthoxy-4 phényl)-3 propionate de méthyle.

Dans un réacteur de 250ml muni d'un système chauffage-refroidissement, d'un agitateur mécanique, d'un thermomètre et d'un réfrigérant,on plce, sous atmosphère inerte, 13,875g (0,111 mole) d'o-aminothiophénol, 20,8g (0,1 mole) de trans(-)(2R,3S) époxy-2,3 (méthoxy-4 phényl)-3 propionate de méthyle et 100ml de toluène.
On porte l'ensemble au reflux durant 2 heures puis on distille le toulène de manière à obtenir 34g d'un résidu solide (rendement massique : 100%).

De cette manière, on obtient le cis(+)(2S,3S) hydroxy-2 (amino-2 phénylthio)-3 (méthoxy-4 phényl)-3 propionate de méthyle qui peut être engagé brut dans l'étape suivante ou que l'on peut recristalliser dans un mélange méthanol/eau de manière à obtenir un composé analytiquement pur.

b) Acide cis(+)(2S,3S) hydroxy-2 (méthoxy-4 phényl)-3 (amino-2 phénylthio)-3 propionate.

Dans un ballon de 250ml muni d'un système de chauffage-refroidissement, d'un agitateur mécanique, d'un thermomètre et d'un réfrigérant, on place, sous atmosphère inerte, 33,1g(0,1 mole) de cis(+)(2S,3S) hydroxy-2 (amino-2 phénylthio)-3 (méthoxy-4 phényl)-3 propionate de méthyle brut et 160ml d'hydroxyde de sodium à 5%. Sous vive agitation, on porte le milieu à 50°C et on l'y maintient durant 2 heures. Le milieu réactionnel est limpide et brun. Après refroidissement à température ambiante, on verse le mélange dans 82ml d'acide chlorhydrique à 9%, ce qui provoque la précipitation du produit attendu. On le filtre et on le lave à l'eau puis on le sèche à l'étuve sous vide à 20°C.

De cette manière, on obtient l'acide cis(+)(2S,3S) hydroxy-2 (méthoxy-4 phényl)-3 (amino-2 phénylthio)-3 propionate sous forme brute que l'on peut purifier par réempâtage dans l'éthanol.
P.F. : 138°C
$\alpha_D^{20}$ : 346°

c) Cis(+)(2S,3S) hydroxy-2 (méthoxy-4 phényl)-2 dihydro-2,3 benzothiazépine (5H)-1,5 one-4.

Dans un réacteur de 21 muni d'un système de chauffage-refroidissement, d'un agitateur mécanique, d'un thermomètre, d'un réfrigérant et d'un système Dean-Stark, on introduit, sous atmosphère inerte, 50g (0,156 mole) d'acide cis(+)(2S,3S) hydroxy-2 (méthoxy-4 phényl)-3 (amino-2 phénylthio)-3 propionate et 940ml de xylène. On porte l'ensemble au reflux et on l'y maintient durant 8 heures. On refroidit à -10°C, on filtre, on rince le solide avec du xylène et on sèche en étuve ventilée.

De cette manière, on obtient 43g de cis(+)(2S,3S) hydroxy-3 méthoxy-4 phényl)-3 dihydro-2,3 benzothiazépine (5H)-1,5 one-4.
Rendement : 91% P.F. : 200°C
$\alpha_D^{20}$ : + 115°.

Le filtrat contient encore 6 à 7% du produit désiré que l'on peut récupérer.

d) Cis(+)(2S,3S) hydroxy-3(N,N-diméthylamino-2 éthyl)-5 (méthoxy-4 phényl)-2 dihydro-2,3 benzothiazépine (5H)-1,5 one-4.

Dans un réacteur de 250ml muni d'un système de chauffage-refroidissement, d'un agitateur mécanique, d'un thermomètre et d'un réfrigérant, on introduit, sous atmosphère inerte, 12g (0,04 mole) de cis(+)(2S,3S) hydroxy-3 (méthoxy-4 phényl)-2 dihydro-2,3 benzothiazépine (5H)-1,5 one-4 et 60ml de N,N-diméthylformamide. Le milieu étant sous agitation, on obtient une solution limpide. On additionne alors 16,6g (0,12 mole) de carbonate de potassium et 6,9g (0,048 mole) de chlorhydrate de diméthylamino-2 chloro-1 éthane. On chauffe l'ensemble à 60°C durant une heure. Après refroidissement du milieu à température ambiante, on filtre les sels minéraux et on concentre sous vide. On reprend dans 75ml de dichloroéthane le résidu huileux obtenu et on lave à l'eau. On concentre la phase organique et on reprend le résidu dans l'éther de pétrole de manière à obtenir la cristallisation du produit attendu. On filtre et on sèche à l'étuve sous vide.

De cette manière, on obtient 13,65g de cis(+)(2S,3S)hydroxy-3 (N,N-diméthylaminoéthyl)-5 (méthoxy-4 phényl)-2 dihydro-2,3 benzothiazépine (5H)-1,5 one-4.
Rendement : 91,6%
P.F. : 86-87 °C
$\alpha_D^{20}$ : + 186,8° (c = 0,25, méthanol).

e) Chlorhydrate de cis(+)(2S,3S) acétoxy-3 (N,N-diméthylamino-2 éthyl)-5 (méthoxy-4 phényl)-2 dihydro-2,3 benzothiazépine (5H)-1,5 one-4.

Dans un ballon tricol de 50ml muni d'un système de chauffage-refroidissement, d'un agitateur mécanique, d'un thermomètre et d'un réfrigérant, on introduit 5,6g (0,015 mole) de cis(+)(2S,3S) hydroxy-3 (N,N-diméthylamino-2 éthyl)-5 (méthoxy-4 phényl)-2 dihydro-2,3 benzothiazépine (5H)-1,5 one-4 et 14ml de toluène sec. On place l'ensemble sous agitation et on additionne 1,7g (0,0165 mole) d'anhydride acétique. On chauffe le milieu à reflux du toluène durant 3 heures puis on refroidit à tempé rature ambiante et on ajoute 3,4ml d'une solution éthanolique d'acide chlorhydrique 4,8N. On refroidit à 0 °C et on filtre le précipité obtenu. On lave au toluène et on sèche.

De cette manière, on obtient 6,25g de chlorhydrate de cis(+) (2S,3S) acétoxy-3 (N,N-diméthylamino-2 éthyl)-5 (méthoxy-4 phényl)-2 dihydro-2,3 benzothiazépine (5H)-1,5 one-4.
Rendement : 92,4%
P.F. : 210 °C
$\alpha_D^{20}$ : + 116,5° (c = 1%, eau).

## Revendications

1. Procédé de préparation du diastéréoisomère trans(-)(2R,3S) des esters glycidiques de formule générale :

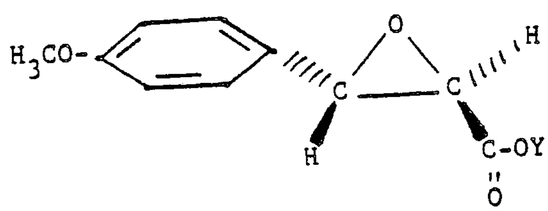

I

dans laquelle Y représente un radical alkyle en $C_1$-$C_8$, caractérisé en ce que l'on fait réagir une chlorhydrine de formule générale :

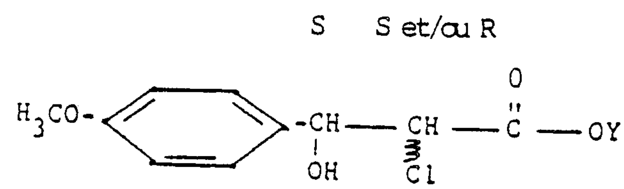

II

dans laquelle Y a la même signification que précédemment, avec le 1,8-diazabicyclo [5,4,0] undec-7ène dans un solvant approprié et à une température comprise entre -10 °C et la température ambiante, pour fournir les composés désirés.

2. Procédé selon la revendication 1, caractérisé en ce que l'on prépare un diastéréoisomère trans(-)(2R,3S) des esters glycidiques de formule générale I dans laquelle Y est choisi parmi un radical méthyle, éthyle, n-propyle, isopropyle, n-butyle, isobutyle, tertiobutyle, n-pentyle, n-hexyle, n-heptyle et n-octyle.

3. Procédé selon la revendication 1 ou 2 caractérisé en ce que le solvant approprié est choisi parmi un solvant aprotique polaire, un solvant apro tique apolaire, un solvant polaire protique et un solvant polaire aprotique.

4. Procédé selon la revendication 3, caractérisé en ce que le solvant est choisi parmi le dichloréthane et un alcool.

5, Procédé selon l'une quelconque des revendications, 1 à 4, caractérisé en ce que la température est

comprise entre -10° C et +10° C.

6. Procédé selon l'une quelconque des revendications 1 à 5, caractérisé en ce que la chlorhydrine de formule II est sous forme de diastéréoisomère anti(+)(2S,3S).

7. Procédé selon l'une quelconque des revendications 1 à 6, caractérisé en ce que la chlorhydrine de formule II est sous forme de diastéréoisomère syn(-)(2R,3S).

8. Procédé selon l'une quelconque des revendications 1 à 7, caractérisé en ce que la chlorhydrine de formule II est sous forme d'un mélange de diastéréoisomères anti(+)(2S,3S) et syn(-)(2R,3S).

9. Chlorhydrines de formule générale

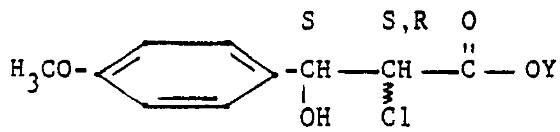

II

dans laquelle Y représente un radical alkyle en $C_1$-$C_8$.

10. Chlorhydrines selon la revendication 9, caractérisées en ce que Y est choisi parmi un radical méthyle, éthyle, n-propyle, isopropyle, n-butyle, isobutyle, tertiobutyle, n-pentyle, n-hexyle, n-heptyle et n-octyle.

11. Chlorhydrines selon la revendication 10, caractérisées en ce que Y représente le radical méthyle.

12. Chlorhydrines selon l'une quelconque des revendications 9 à 11, caractérisées en ce qu'elles sont sous forme de diastéréoisomères anti(+)(2S,3S).

13. Chlorhydrines selon l'une quelconque des revendications 9 à 11, caractérisées en ce qu'elles sont sous forme de diastéréoisomères syn(-)(2R,3S).

14. Chlorhydrines selon l'une quelconque des revendications 9 à 11, caractérisées en ce qu'elles sont sous forme de mélanges de diastéréoisomères anti(+)(2S,3S) et syn(-)(2R,3S).

15. Anti(+)(2S,3S) chloro-2 hydroxy-3 (méthoxy-4 phényl)-3 propionate de méthyle.

16. Syn(-)(2R,3S) chloro-2 hydroxy-3 (méthoxy-4 phényl)-3 propionate de méthyle.

17. Mélanges d'anti(+)(2S,3S) et syn(-)(2R,3S) chloro-2 hydroxy-3 (méthoxy-4 phényl)-3 propionates de méthyle.

18. Procédé de préparation de dérivés diastéréoisomères cis(+)(2S,3S) de benzothiazépine-1,5 one-4 de formule générale :

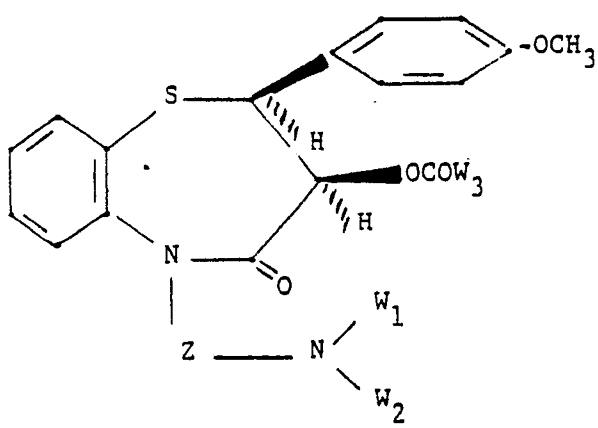

I a

dans laquelle $W_1$, $W_2$ et $W_3$, identiques ou différents, représentent chacun un radical alkyle en $C_1$-$C_4$ et Z représente un radical alkylène en $C_1$-$C_4$, caractérisé en ce que :

a) on fait réagir une chlorhydrine de formule générale:

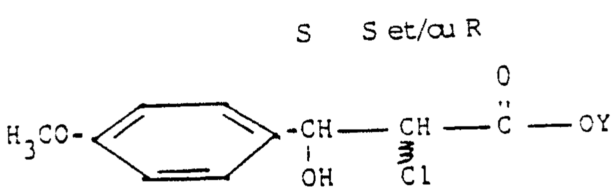

II

dans laquelle Y a la même signification que dans la revendication 1, avec le 1,8-diazabicyclo [5,4,0] undec-7ène dans un solvant approprié et à une température comprise entre -10 °C et la température ambiante pour former les diastéréoisomères trans(-)(2R,3S) des esters glycidiques de formule générale :

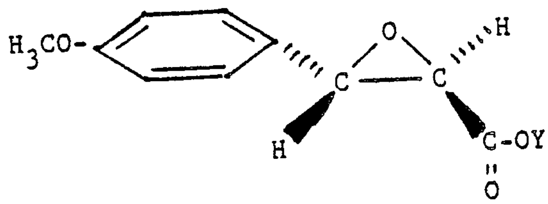

dans laquelle Y a la même signification que précédemment,

b) on fait réagir le diastéréoisomère trans(-)(2R,3S) ainsi obtenu avec l'amino-2 thiophénol pour donner un ester cis(+)(2S,3S) hydroxy-2 (méthoxy-4 phényl)-3 (amino-2 phénylthio)-3 propionique de formule générale :

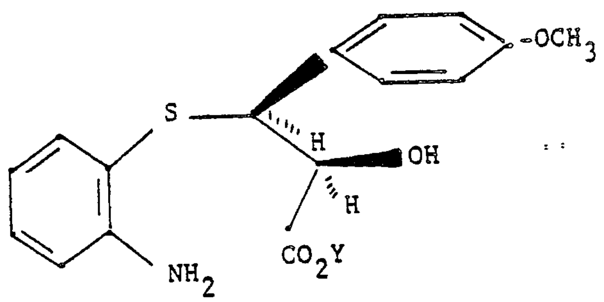

dans laquelle Y a la même signification que précédemment,

c) on saponifie l'ester cis(+)(2S,3S) hydroxy-2 (méthoxy-4 phényl)-3 (amino-2 phénylthio)-3 propionique ainsi obtenu au moyen d'un agent basique, pour obtenir l'acide cis(+)(2S,3S) hydroxy-2 (méthoxy-4 phényl)-3 (amino-2 phénylthio)-3 propionique,

d) on cyclise par chauffage à une température allant de 100 à 140 °C dans un hydrocarbure aromatique, l'acide cis(+)(2S,3S) hydroxy-2 (méthoxy-4 phényl)-3 (amino-2 phénylthio)-3 propionique ainsi obtenu, ce qui fournit la cis(+)(2S,3S) hydroxy-3 (méthoxy-4 phényl)-2 dihydro-2,3 benzothiazépine (5H)-1,5 one-4,

e) on fait réagir ce dérivé cis(+)(2S,3S) de benzothiazépine avec un halogénure de formule générale :

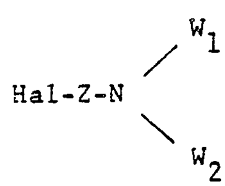

dans laquelle Hal représente un atome d'halogène et $W_1$, $W_2$ et Z ont la même signification que précédemment, ou un sel de cet halogénure, en présence d'un agent basique et dans un solvant polaire, pour obtenir les dérivés cis(+)(2S,3S) de benzothiazépine-1,5 one-4 de formule générale :

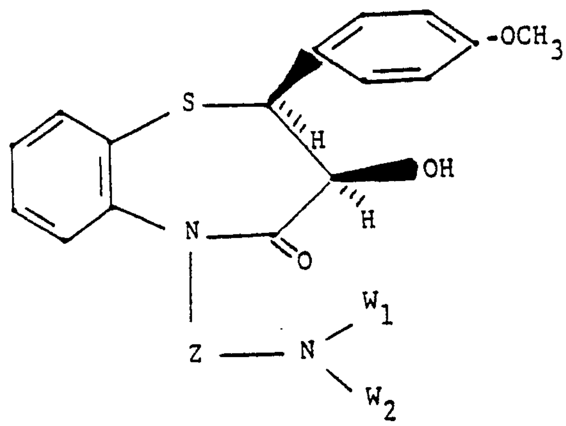

dans laquelle $W_1$, $W_2$ et Z ont la même signification que précédemment,
f) on fait réagir, dans un solvant aprotique au reflux, le dérivé cis(+)(2S,3S) de benzothiazépine-1,5 one-4 ainsi obtenu avec un agent d'acylation approprié choisi parmi un anhydride de formule générale :

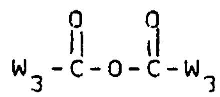 et un acide

de formule générale 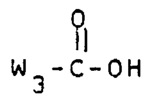

dans lesquelles $W_3$ représente un radical alkyle en $C_1$-$C_4$, pour obtenir les dérivés diastéréoisomères cis(+)(2S,3S) de benzothiazépine-1,5 one-4 désirés, que l'on peut faire réagir, si on le désire, avec un acide organique ou inorganique approprié pour obtenir un sel pharmaceutiquement acceptable de ce dérivé.

19. Procédé selon la revendication 18, caractérisé en ce que l'on prépare le diastéréoisomère cis(+)(2S,3S) de formule Ia dans laquelle $W_1$, $W_2$ et $W_3$ représentent chacun méthyle et Z représente éthylène.

Office européen des brevets

# RAPPORT DE RECHERCHE EUROPEENNE

Numero de la demande

EP 90 40 2044

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | GB-A-2 139 620 (SHIONOGI)<br>* En entier * | 1-19 | C 07 D 303/48<br>C 07 C 69/732<br>C 07 D 281/10 |
| D,Y | TETRAHEDRON LETTERS, vol. 27, no. 44, 1986, pages 5397-5400, Pergamon Journals Ltd, GB; H. AKITA et al.: "Determination of absolute structure of (-)-oudemansin B"<br>* En entier * | 1-19 | |
| Y | JOURNAL OF THE CHEMICAL SOCIETY, PERKIN TRANSACTIONS II, no. 10, 1973, pages 1480-1490; P.B.D. DE LA MARE et al.: "The kinetics and mechanisms of additions of olefinic substances. Part XL. Stereochemistry of addition of chlorine acetate and of chlorine to some unsaturated compounds"<br>* En entier, et en particulier page 1482, tableau 2, composé 3 * | 1-19 | |
| P,Y | CHEMICAL ABSTRACTS, vol. 112, no. 17, 23 avril 1990, page 698, résumé no. 158034e, Columbus, Ohio, US; & JP-A-01 226 881 (TANABE SEIYAKU CO., LTD) 11-09-1989 | 1-19 | |

| DOMAINES TECHNIQUES RECHERCHES (Int. Cl.5) |
|---|
| C 07 D 303/00<br>C 07 C 69/00<br>C 07 D 281/00 |

Le présent rapport a été établi pour toutes les revendications

| Lieu de la recherche | Date d'achèvement de la recherche | Examinateur |
|---|---|---|
| LA HAYE | 02-10-1990 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant